(19) Europäisches Patentamt
European Patent Office
Office européen des brevets

(11) EP 0 701 989 B1

(12) **FASCICULE DE BREVET EUROPEEN**

(45) Date de publication et mention de la délivrance du brevet:
**28.10.1998 Bulletin 1998/44**

(51) Int Cl.[6]: **C07C 51/285**, C07C 51/27

(21) Numéro de dépôt: **95401942.8**

(22) Date de dépôt: **24.08.1995**

(54) **Procédé de préparation d'acides mono- et dicarboxyliques à partir d'acides gras insaturés et/ou leurs dérivés**

Verfahren zur Herstellung von Mono- und Dicarbonsäuren aus ungesättigten Fettsäuren und/oder ihren Derivaten

Process for preparing mono- and dicarboxylic acids from unsaturated fatty acids and/or their derivatives

(84) Etats contractants désignés:
**AT BE CH DE DK ES FR GB GR IE IT LI NL PT SE**

(30) Priorité: **16.09.1994 FR 9411050**

(43) Date de publication de la demande:
**20.03.1996 Bulletin 1996/12**

(73) Titulaire: **NOVANCE S.A.**
**60206 Compiegne (FR)**

(72) Inventeurs:
- **Dos Santos, Emmanuel**
**F-69320 Feyzin (FR)**
- **Metivier, Pascal**
**F-69110 Sainte Foy les Lyon (FR)**

(74) Mandataire: **Dubruc, Philippe et al**
**RHODIA SERVICES**
**Direction de la Propriété Industrielle**
**25, quai Paul Doumer**
**92408 Courbevoie Cédex (FR)**

(56) Documents cités:
**FR-A- 1 400 437**
**FR-A- 2 223 349**
**US-A- 2 773 095**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 701 989 B1

## Description

La présente invention a pour objet un procédé de préparation d'acides carboxyliques, à partir d'acides gras insaturés et/ou leurs dérivés.

Plus précisément, l'invention concerne un procédé de préparation d'acides carboxyliques aliphatiques, monofonctionnels et bifonctionnels, à partir d'acides gras insaturés sous forme d'acide ou d'ester, et plus particulièrement sous forme de triglycéride.

Divers procédés ont été proposés pour réaliser la préparation des acides carboxyliques, à partir d'acides gras insaturés.

Un procédé relativement ancien et décrit dans US-A-2 773 095 consiste à effectuer l'oxydation des ces derniers, en les soumettant à une oxydation à l'aide d'acide nitrique dilué et sous pression. L'oxydation de l'acide oléique conduit à l'obtention de l'acide pélargonique et de l'acide azélaïque. Toutefois, la sélectivité de la réaction conduite selon US-A-2 773 095 est mauvaise car l'on note une suroxydation conduisant à la formation de monoacides et de diacides inférieurs.

Par ailleurs, il est connu selon US-2 865 937 de préparer des acides mono- et dicarboxyliques, à partir d'acides gras insaturés, notamment d'acide oléique, en soumettant le substrat de départ à une coupure par ozonolyse suivie d'une décomposition oxydante. Un inconvénient majeur de ce type de procédé est son coût prohibitif en raison de la mise en oeuvre de l'ozone.

Un autre procédé également coûteux est décrit dans FR-A 2 101 729. Un exemple de préparation mentionne l'oxydation de l'acide oléique par le peroxyde d'hydrogène, suivie par une hydrolyse à l'acide sulfurique, d'une séparation par solvant et d'une autre étape d'oxydation effectuée par un acide percarboxylique, en présence d'ions métalliques. Outre le nombre étapes décrites, il y a lieu de noter l'importance du coût des réactifs engagés.

Tous ces procédés ne donnent pas satisfaction car ils sont difficilement transposables à l'échelle industrielle, soit en raison de mauvais rendements réactionnels, soit pour des raisons économiques.

L'objet de la présente invention est de fournir un procédé permettant d'obvier aux inconvénients précités.

Il a maintenant été trouvé et c'est ce qui constitue l'objet de la présente invention un procédé de préparation d'acides mono- et dicarboxyliques, à partir d'acides gras insaturés et/ou leurs dérivés caractérisé par le fait qu'il comporte les étapes suivantes :

- une étape qui consiste à oxyder un acide gras insaturé sous forme d'acide et/ou d'ester par le peroxyde d'hydrogène, en présence d'un oxyde métallique ou d'un acide carboxylique éventuellement associé à un catalyseur à base de ruthénium,
- une étape qui consiste à faire réagir le milieu réactionnel issu de l'étape précédente, avec de l'acide nitrique en présence d'un catalyseur à base de vanadium, éventuellement associé à un co-catalyseur,
- puis une étape de récupération des acides mono- et dicarboxyliques.

Le procédé de l'invention comporte une première opération d'oxydation ménagée du substrat de départ permettant ainsi de limiter la consommation en peroxyde d'hydrogène conduisant à un produit intermédiaire dans lequel les doubles liaisons sont oxydées en pont époxy et/ou en deux groupements hydroxyle vicinaux.

Sans séparation du produit intermédiaire, on enchaîne une étape de coupure et d'hydrolyse oxydante effectuée par l'acide nitrique permettant ainsi d'obtenir les acides mono- et dicarboxyliques souhaités.

Le procédé de l'invention est particulièrement intéressant car il permet d'obtenir lesdits acides, avec une bonne sélectivité de réaction.

De plus, il induit un coût de réactifs tout à fait compatible avec une exploitation industrielle.

Enfin, un autre avantage et non des moindres, est qu'il peut être mis en oeuvre non seulement avec des acides gras insaturés, par exemple l'acide oléique mais également à partir des acides gras insaturés sous forme de triglycérides, ce qui permet de partir directement d'huiles ou de graisses les contenant.

Le procédé de l'invention permet ainsi de valoriser les huiles, notamment les huiles de colza et de tournesol qui peuvent être disponibles sur le marché en quantité importante.

Le procédé de l'invention s'applique à tout acide gras présentant au moins une double liaison.

Il peut être symbolisé par la formule (I) :

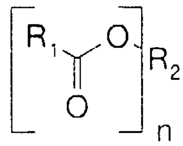

(I)

dans laquelle :

- n est un nombre égal à 1, 2 ou 3,
- $R_1$ représente un radical alcényle ou alcadiényle, linéaire ou ramifié ayant de 4 à 40 atomes de carbone,
- $R_2$ peut prendre les significations suivantes :
  - si n = 1, $R_2$ représente un atome d'hydrogène, ayant de préférence de 1 à 6 atomes de carbone, éventuellement porteur d'un ou plusieurs groupements hydroxyle,
  - si n = 2, $R_2$ représente un radical alkylène ou alcénylène, ayant de préférence de 1 à 6 atomes de carbone, éventuellement porteur d'un ou plusieurs groupements hydroxyle,
  - si n = 3, $R_2$ représente un radical alkyle ayant 3 atomes de carbone.

Les composés mis en oeuvre préférentiellement répondent à la formule (I) dans laquelle $R_1$ représente un radical alcényle ayant de 6 à 22 atomes de carbone et présentant de 1 à 5 doubles liaisons, de préférence 1 à 3 et $R_2$ représente un atome d'hydrogène ou un radical alkyle ayant de 1 à 4 atomes de carbone porteur éventuellement de 1 à 2 groupes hydroxyle, pour n = 1 ; un radical diéthylène ou propylène éventuellement porteur d'un groupe hydroxyle pour n = 2 ; un radical propanetriyle-1,2,3 pour n = 3.

Comme exemples d'acides gras insaturés, on peut citer les acides gras insaturés présentant une seule double liaison tel que l'acide lindérique, l'acide myristoléique, l'acide palmitoléique, l'acide oléique, l'acide pétrosélénique, l'acide doeglique, l'acide gadoléique, l'acide érucique ; les acides gras insaturés présentant deux doubles liaisons tels que l'acide linoléique ; les acides gras insaturés présentant 3 doubles liaisons tels que l'acide linolénique ; les acides gras insaturés présentant plus de 4 doubles liaisons tels que l'acide isanique, l'acide stéarodonique, l'acide arachidonique, l'acide chypanodonique ; les acides gras insaturés porteurs de groupe hydroxyle tel que l'acide ricinoléique ainsi que leurs mélanges.

Parmi les acides précités, on met en oeuvre préférentiellement les acides gras suivants : l'acide palmitoléique, l'acide oléique, l'acide pétrosélénique, l'acide érucique, l'acide linoléique, l'acide linolénique, l'acide ricinoléique.

Une source naturelle des acides gras sont les huiles et les graisses.

En effet, le procédé de l'invention est particulièrement intéressant car il permet de partir des huiles et des graisses naturelles qui sont des esters de glycérol. Elles contiennent des mélanges de triglycérides d'acides gras généralement en mélange avec des acides gras saturés.

Comme sources d'origine animale, on peut citer en autres, l'huile de cachalot, l'huile de dauphin, l'huile de baleine, l'huile de phoque, l'huile de sardine, l'huile de hareng, l'huile de squale, l'huile de foie de morue ; l'huile de pied de boeuf, les graisses de boeuf, de porc, de cheval, de mouton (suifs).

A titres d'exemples de sources d'origine végétale, on peut mentionner, entre autres, l'huile de colza, l'huile de tournesol, l'huile d'arachide, l'huile d'olive, l'huile de noix, l'huile de maïs, l'huile de soja, l'huile de lin, l'huile de chanvre, l'huile de pépins de raisin, l'huile de coprah, l'huile de palme, l'huile de graines de coton, l'huile de babassu, l'huile de jojoba, l'huile de sésame, l'huile de ricin.

Les huiles mentionnées ci-après sont mises en oeuvre préférentiellement dans le procédé de l'invention : l'huile de colza, l'huile de tournesol, l'huile de soja, l'huile de lin, l'huile de ricin.

Il est également possible de partir des esters correspondants auxdits acides, en particulier les esters méthylique, éthylique et propylique et l'on peut citer plus particulièrement les produits de méthanolyse notamment des huiles, et plus préférentiellement des huiles de colza.

Dans l'exposé qui suit de la présente invention, on utilisera le terme "acide gras insaturé" de manière générique et il désignera aussi bien les acides gras insaturés à proprement parler, seuls ou en mélange ainsi que leur forme ester ou triglycéride.

Conformément au procédé de l'invention, on effectue l'oxydation du substrat insaturé, par le peroxyde d'hydrogène.

Le peroxyde d'hydrogène mis en oeuvre selon l'invention peut être sous forme de solution aqueuse.

La concentration de la solution aqueuse de peroxyde d'hydrogène est généralement d'au moins 20 % en poids de $H_2O_2$ et, de préférence, comprise entre 20 et 70 % en poids.

La quantité de peroxyde d'hydrogène mise en oeuvre est fonction du nombre de doubles liaisons à oxyder présentes dans le substrat de départ. Généralement, on met en oeuvre une quantité égale à la stoechiométrie ou un léger excès allant de 0 à 50 %, de préférence, compris entre 1 et 20 %.

La première étape du procédé de l'invention peut être conduite selon plusieurs variantes qui diffèrent par ce qui est associé au peroxyde d'hydrogène.

Un mode de réalisation de l'invention consiste à ajouter au peroxyde d'hydrogène, un acide carboxylique permettant ainsi la formation in situ d'un peracide.

Les acides carboxyliques susceptibles d'être mis en oeuvre peuvent être mono- ou polycarboxyliques. Ce sont des composés ne présentant pas d'insaturations.

Ils répondent plus particulièrement à la formule générale suivante (II) :

$$R_3 - COOH \qquad (II)$$

dans ladite formule (II), $R_3$ représente un radical hydrocarboné ayant de 1 à 22 atomes de carbone qui peut être un radical aliphatique acyclique saturé, linéaire ou ramifié, un radical cycloaliphatique saturé, monocyclique ou polycyclique.

Ledit radical $R_3$ peut porter un autre groupe fonctionnel COOH. Il peut également porter d'autres substituants, par exemple, alkoxy ou halogène dans la mesure où ils n'interfèrent pas dans la réaction.

$R_3$ représente plus particulièrement un radical alkyle linéaire ou ramifié ayant de 1 à 22 atomes de carbone ou un radical cycloalkyle ayant de 5 à 7 atomes de carbone.

Comme exemples d'acides carboxyliques susceptibles d'être mis en oeuvre dans le procédé de l'invention, on peut citer : les acides monocarboxyliques aliphatiques tels que l'acide formique, l'acide acétique, l'acide propionique, l'acide butyrique, l'acide valérique, l'acide hexanoïque ; les acides dicarboxyliques aliphatiques tels que l'acide succinique, l'acide adipique ; les acides cycloaliphatiques tels que l'acide cyclopentane carboxylique, l'acide cyclohexane carboxylique.

Parmi les acides carboxyliques précités, on fait appel préférentiellement aux acides carboxyliques, aliphatiques saturés de préférence, à l'acide formique ou l'acide acétique.

Il est également intéressant de mettre en oeuvre un acide carboxylique qui se retrouve dans les produits formés au cours du procédé de l'invention et l'on peut citer plus particulièrement, l'acide pélargonique, l'acide azélaïque et l'acide stéarique.

On ne sortira pas du cadre de la présente invention, à mettre en oeuvre soit directement un acide percarboxylique au lieu de le préparer in situ, soit un précurseur d'acide carboxylique qui conduirait dans les conditions réactionnelles oxydantes, à ce dernier.

La quantité d'acide carboxylique mise en oeuvre peut varier dans de larges limites. Elle peut aller d'une quantité catalytique à une quantité stoechiométrique.

Plus précisément, le rapport molaire entre le peroxyde d'hydrogène et l'acide carboxylique varie entre 10 et 500 %, de préférence entre 10 et 40 %.

Selon une variante modifiée, il est également possible selon l'invention d'effectuer l'oxydation de l'acide gras insaturé, par le peroxyde d'hydrogène, en présence d'acide carboxylique et d'un catalyseur à base de ruthénium.

On peut faire appel, comme catalyseur, à tout composé du ruthénium.

A titre d'exemples de composés susceptibles d'être mis en oeuvre en tant que catalyseur de l'invention, on peut citer, entre autres, le chlorure de ruthénium III, le chlorure de ruthénium IV, le pentafluorure de ruthénium, l'oxyde de ruthénium II, l'oxyde de ruthénium IV, l'oxychlorure de ruthénium ammoniaqué $Ru_2(OH)_2Cl_4$, $7NH_3$, $5H_2O$, l'acétate de ruthénium.

On choisit préférentiellement, comme catalyseur à base de ruthénium, le chlorure de ruthénium III.

La quantité de catalyseur à base de ruthénium mise en oeuvre, exprimée par le rapport pondéral entre le peroxyde d'hydrogène et le catalyseur peut être avantageusement comprise entre 1 et 35 %, de préférence entre 3 et 10 %.

Une autre variante d'exécution de la première étape d'oxydation ménagée intervenant dans le procédé de l'invention, consiste à effectuer l'oxydation de l'acide gras insaturé par le peroxyde d'hydrogène, en présence d'un catalyseur à base d'un métal du groupe VIa de la classification périodique des éléments.

Pour la définition des éléments, on se réfère ci-après à la Classification périodique des éléments publiée dans le Bulletin de la Société Chimique de France, n°1 (1966).

On fait appel préférentiellement à un catalyseur à base de tungstène et/ou de molybdène.

Le catalyseur est choisi de préférence parmi : l'acide tungstique, l'acide phosphotungstique, l'acide molybdique, l'acide phosphomolybdique.

Il est donc, de préférence, sous forme acide. Il est possible de partir directement des composés précités ou bien de les former in situ à partir de leurs oxydes ou sels.

Comme exemples de catalyseurs à base de molybdène, on peut citer notamment :

- les halogénures de molybdène, par exemple, l'hexafluorure de molybdène, le tri- tétra- ou pentachlorure de molybdène, le di- tri- ou tétrabromure de molybdène,
- les hydroxydes de molybdène $Mo(OH)_3$, $MoO(OH)_3$ ou $Mo_2O_3$, $3H_2O$,
- les oxydes de molybdène tels que le dioxyde, le trioxyde, le pentaoxyde ou le sesquioxyde de molybdène,
- les oxyhalogénures de molybdène tels que l'oxydifluorure ou l'oxytétrafluoure de molybdène, l'oxydichlorure, l'oxytrichlorure, l'oxytétrachlorure, l'oxypentachlorure de molybdène, l'oxychlorure acide de molybdène, l'oxydibromure de molybdène,
- le métaphosphate de molybdène,
- le phosphomolybdate d'ammonium.
- le molybdate d'ammonium.

Pour ce qui est du catalyseur au tungstène, on peut partir plus particulièrement :

- les halogénures de tungstène, par exemple, l'hexafluorure de tungstène, le di- tétrapentaou hexachlorure de tungstène, le di- penta- ou hexabromure de tungstène,
- les oxydes de tungstène tels que le dioxyde, le trioxyde, le pentaoxyde ou le sesquioxyde de tungstène,
- les oxyhalogénures de tungstène tels que l'oxytétrafluoure de tungstène, l'oxydichlorure, l'oxytétrachlorure de tungstène, l'oxydibromure, l'oxytétrabromure de tungstène,
- le métaphosphate de tungstène,
- le tungstate d'ammonium,
- le phosphotungstate d'ammonium.

La quantité de catalyseur à base de tungstène et/ou molybdène mise en oeuvre, exprimée par le rapport pondéral entre le peroxyde d'hydrogène et le catalyseur peut être avantageusement comprise entre 1 et 35 %, de préférence entre 3 et 10 %.

La formation des catalyseurs sous forme acide se fait lorsque l'on part des composés précités, par ajout d'une faible quantité d'acide fort.

Par acide fort, on désigne dans la présente invention, un acide présentant un pKa dans l'eau inférieur à - 0,1 et, de préférence, inférieur à - 1,0.

Le pKa est défini comme la constante de dissociation ionique du couple acide/base, lorsque l'eau est utilisée comme solvant.

On peut citer plus particulièrement les oxyacides halogénés ou non tels que l'acide nitrique, l'acide sulfurique, l'acide pyrosulfurique, l'acide phosphorique, les acides polyphosphoriques, l'acide perchlorique, les acides halogénosulfoniques tels que l'acide fluorosulfonique, l'acide chlorosulfonique ou l'acide trifluorométhanesulfonique, l'acide méthanesulfonique, l'acide éthane-sulfonique, l'acide éthanedisulfonique, l'acide benzènesulfonique, les acides benzènedisulfoniques, les acides toluènesulfoniques, les acides naphtalène-sulfoniques et les acides naphtalènedisulfoniques.

Parmi ces acides, on choisit, tout particulièrement, l'acide nitrique, l'acide sulfurique ou l'acide phosphorique.

La quantité d'acide fort mise en oeuvre, exprimée par le rapport pondéral entre l'acide fort et le peroxyde d'hydrogène peut être avantageusement comprise entre 1 et 50 %, de préférence entre 5 et 20 %.

On effectue la première étape d'oxydation à une température avantageusement comprise entre 30 et 100°C, de préférence entre 60°C et 100°C.

Le procédé de l'invention est généralement mis en oeuvre sous pression atmosphérique mais il peut l'être également sous des pressions supérieures ou inférieures à la pression atmosphérique.

D'un point de vue pratique, le procédé de l'invention est facile à mettre en oeuvre. On charge les différents réactifs. Il n'y a pas d'ordre d'introduction à respecter seulement on préfère additionner le peroxyde d'hydrogène, par addition progressive, en continu ou par fractions dans un mélange réactionnel contenant le substrat, l'acide carboxylique et les catalyseurs si nécessaires.

En fin de réaction, on obtient un produit intermédiaire dans lequel les doubles liaisons sont oxydées en pont époxy et/ou en deux groupements hydroxyle vicinaux.

Selon le procédé de l'invention, on n'isole pas le produit intermédiaire et l'on enchaîne directement l'étape suivante de coupure et d'hydrolyse oxydante effectuée par l'acide nitrique.

On fait appel à une solution aqueuse d'acide nitrique ayant une concentration indifférente pouvant varier entre 30 % et 100 %. Toutefois, une concentration comprise entre 40 % et 60 % est préférée.

L'acide nitrique est mis en jeu, en large excès. La quantité d'acide nitrique représente de 2 à 50 fois le poids du substrat de départ et plus préférentiellement de 4 à 10 fois.

Il est préférable afin d'amorcer l'oxydation nitrique d'adjoindre un générateur de NO+. Ainsi, on peut partir du dioxyde d'azote $NO_2$, de l'anhydride azoteux $N_2O_3$, du peroxyde d'azote $N_2O_4$, de l'oxyde d'azote NO. Dans le cas où ledit agent est gazeux dans les conditions réactionnelles, on le fait buller dans le milieu.

On peut également faire appel à l'acide nitreux, à un sulfate de nitrosyle ou nitrose ou à un sel nitreux, de préférence un sel de métal alcalin, et encore plus préférentiellement, de sodium.

La quantité de cet agent peut varier largement. Elle est avantageusement comprise entre 0 et 5 % du poids d'acide nitrique et de préférence, entre 0 et 1 %.

Comme mentionné précédemment, l'acide nitrique est utilisé en présence d'un catalyseur à base de vanadium.

On peut utiliser l'un des composés énoncés ci-après, à titre de catalyseurs :

- les halogénures de vanadium tels que le tri- tétra- ou pentafluorure de vanadium, le di- tri- ou tétrachlorure de vanadium, le tribromure de vanadium,
- les oxydes de vanadium tels que l'oxyde de vanadium, le dioxyde de vanadium, le sesquioxyde de vanadium, le pentaoxyde de vanadium,
- les oxyhalogénures de vanadium en particulier les oxy di- ou trifluorure de vanadium, les oxy mono- di- ou trichlorure de vanadium, les oxy mono- di- ou tribromure de vanadium,
- le sulfate de vanadium,
- le sulfate de vanadyle,
- les vanadates de métaux alcalins ou d'ammonium sous forme ortho- méta- ou pyro,
- l'acétylacétonate de vanadyle.

Cette liste ne présente aucun caractère limitatif et l'on peut aussi faire appel aux sels doubles comprenant du vanadium.

Parmi les composés précités, on choisit préférentiellement les vanadates d'ammonium, comme catalyseur.

La quantité de catalyseur à base de vanadium mise en oeuvre, exprimée par le rapport pondéral entre le catalyseur exprimée en $HVO_3$ et l'acide nitrique est comprise de préférence, entre 0,001 et 1 %, de préférence entre 0,02 et 0,5 %.

Comme mentionné précédemment, il est possible d'ajouter lors de cette oxydation nitrique conduite en présence d'un catalyseur à base de vanadium, un co-catalyseur métallique dont le rôle est d'accélérer la vitesse réactionnelle.

On fait appel à un co-catalyseur à base d'un métal du groupe VIIa et VIII de la classification périodique des éléments et l'on peut citer, préférentiellement, les catalyseurs à base de : manganèse, fer, nickel, ruthénium et cobalt.

A titre d'exemples de composés susceptibles d'être mis en oeuvre comme co-catalyseurs, on peut utiliser, entre autres, les oxydes, hydroxydes, nitrates, halogénures, oxyhalogénures, phosphates, pyrophosphates, carbonates, carboxylates, alcoolates des différents métaux précités.

Les sels préférés sont les suivants : le nitrate de fer II, le nitrate de fer III, le nitrate de nickel II, le nitrate de cobalt II, le nitrate de cobalt III, l'acétate de cobalt II, le carbonate de manganèse II, le chlorure de ruthénium III.

La quantité de co-catalyseur exprimée par le rapport pondéral entre le co-catalyseur et l'acide nitrique est comprise de préférence, entre 0,001 et 1 %, de préférence entre 0,02 et 0,5 %.

Un mode de réalisation préférentielle de l'invention consiste à effectuer cette étape d'oxydation nitrique, en présence d'oxygène ou un gaz en contenant.

Il est ainsi possible, tout au long de la réaction, de régénérer l'acide nitrique à partir de l'acide nitreux.

Ce gaz peut être de l'oxygène pur ou un gaz en contenant, de préférence, l'air. Dans le cas de l'oxygène pur, on peut le faire buller dans le mélange réactionnel à un débit, par exemple, de 0,1 à 50 litres/heure.

La quantité d'oxygène à mettre en oeuvre n'est pas critique dans la mesure où elle est telle que ni les gaz d'alimentation, ni une éventuelle phase gazeuse susceptible d'apparaître dans la zone réactionnelle se trouve dans la plage des compositions explosives, compte tenu des autres paramètres ou conditions réactionnelles choisies. La quantité d'oxygène peut être en excès ou en défaut par rapport à la stoechiométrie de la réaction, vis-à-vis du substrat à oxyder.

Généralement, elle est déterminée en fonction du nombre de doubles liaisons à oxyder. Elle est de 1 à 2 fois la quantité stoechiométrique lorsque le substrat de départ est un acide gras insaturé et de 5 à 8 fois la quantité stoechiométrique quand la matière première est une huile ou une graisse.

La pression d'oxygène ou d'air de la réaction varie entre 1 et 10 bar.

Le procédé de l'invention peut être conduit sous pression atmosphérique.

Dans cette deuxième étape, la température de la réaction est choisie de préférence, entre 40°C et 100°C, et plus préférentiellement entre 60 et 90°C.

Comme précédemment, la réaction est conduite avantageusement sous pression atmosphérique.

La réalisation pratique de l'invention se fait aisément.

Selon un mode préféré, on met le catalyseur et éventuellement les co-catalyseurs dans la solution d'acide nitrique que l'on introduit ensuite dans le mélange réactionnel issu de l'étape précédente.

On fait ensuite buller le courant gazeux comprenant l'oxygène.

En fin de réaction, le milieu réactionnel est bi-phasique.

La phase aqueuse comprend l'acide nitrique, les différents catalyseurs, co-catalyseurs et les acides carboxyliques qui sont majoritairement les acides saturés dicarboxyliques.

La phase organique comprend essentiellement les acides gras saturés monocarboxyliques formés au cours de la réaction et éventuellement ceux présents au départ ainsi que les acides gras insaturés qui n'ont pas réagi.

Il est à noter que le procédé de l'invention présente l'avantage de ne pas dégrader les acides gras saturés qui peuvent être présents dans le substrat de départ, notamment dans le cas des huiles et des graisses.

On procède à la séparation des phases aqueuse et organique, de préférence par décantation à chaud.

On effectue, de préférence, un ou plusieurs lavages de la phase organique, à l'eau afin de récupérer d'éventuels acides gras saturés dicarboxyliques restant en phase organique. Les eaux de lavage peuvent être regroupées avec la phase aqueuse obtenue à la séparation des phases organique et aqueuse.

On refroidit la phase aqueuse contenant les acides gras saturés dicarboxyliques en ramenant la température, à la température ambiante.

Lesdits acides carboxyliques cristallisent et on les récupère selon les techniques classiques de séparation solide/ liquide, de préférence par filtration.

La séparation peut être éventuellement suivie d'un ou plusieurs lavages à l'eau.

Par ailleurs, on traite la phase organique afin d'éliminer le solvant organique par distillation.

On sépare les acides monocarboxyliques de la phase organique selon les techniques classiques de séparation liquide/liquide, de préférence la distillation.

La phase aqueuse qui comprend l'acide nitrique et les catalyseurs et co-catalyseurs peut être avantageusement recyclée, après évaporation de l'eau excédentaire afin d'obtenir la concentration en acide nitrique adéquate.

Le procédé de l'invention est parfaitement bien adapté à la préparation de l'acide pélargonique et l'acide azélaïque obtenus à partir de l'acide oléique et les huiles de colza et de tournesol.

On donne ci-après des exemples de réalisation pratique de l'invention.

Les exemples qui suivent, illustrent l'invention sans toutefois la limiter.

Dans les exemples, les rendements mentionnés correspondent à la définition suivante :

- sélectivité en acide azélaïque : RT

$$RT = \frac{\text{nombre de moles d'acide azélaïque formées}}{\text{nombre de moles d'acide azélaïque + subérique + pimélique formées}}$$

- sélectivité en acide pélargonique : RT

$$RT = \frac{\text{nombre de moles d'acide pélargonique formées}}{\text{nombre de moles d'acides pélargonique + caprylique + heptanoïque formées}}$$

<u>EXEMPLE 1:</u>

Dans cet exemple, on effectue la coupure oxydante de l'huile de tournesol "oléique" en présence d'acide formique et d'acide sulfurique.

Dans un réacteur agité muni d'un réfrigérant et d'une sonde de température, on charge 828 mg d'huile de tournesol "oléique".

La composition en acides gras de cette huile de tournesol, est la suivante :

. acide palmitique : 4,6 %
. acide stéarique : 4,8 %
. acide oléique : 77,8 %
. acide linoléique : 12,8 %

On ajoute 413 mg d'une solution aqueuse d'eau oxygénée à 30 % soit 3,64 mmol, 5 g d'acide formique et 250 mg d'acide sulfurique à 96 %.

Le milieu réactionnel est maintenu 2 heures à 45°C, puis 4 heures à 80°C.

On ajoute ensuite doucement sur ce milieu réactionnel un mélange réactionnel à base d'acide nitrique et composé de 13,2 ml d'une solution aqueuse d'acide nitrique à 49 %, 106 mg de métavanadate d'ammonium et 50 mg de nitrite de sodium. Le milieu réactionnel est maintenu alors 6 heures à 75°C.

En fin de réaction, le milieu réactionnel est refroidi.

Le pH est alors ramené à 2,4 par ajout d'une solution aqueuse de soude 36 %.

Le mélange est alors amené à 500 ml et dosé par électrophorèse capillaire.

Les résultats obtenus sont les suivants :

- acide pélargonique : 236 mg
- acide caprylique : 42 mg
- acide heptanoïque : 11 mg
- acide azélaïque : 117 mg
- acide subérique : 13 mg
- acide pimélique : 12 mg

La sélectivité en acide azélaïque est de 82 %.

La sélectivité en acide pélargonique est de 82 %.

EXEMPLE 2 :

Dans cet exemple, on effectue la coupure oxydante de l'huile de colza "oléique" en présence d'acide formique et d'acide sulfurique.

Dans un réacteur agité muni d'un réfrigérant et d'une sonde de température, on charge 830 mg d'huile de colza "oléique".

La composition en acides gras de cette huile de colza, est la suivante :

- acide palmitique : 3,2 %
- acide stéarique : 2 %
- acide oléique : 86,8 %
- acide linoléique : 2,3 %
- acide linolénique : 4,2 %
- acide gadoléique : 1,5 %

On ajoute 430 mg d'une solution aqueuse d'eau oxygénée à 30 % soit 3,79 mmol, 5 g d'acide formique et 250 mg d'acide sulfurique à 96 %.

Le milieu réactionnel est maintenu 2 heures à 45°C, puis 4 heures à 80°C.

On ajoute ensuite doucement sur ce milieu réactionnel un mélange réactionnel à base d'acide nitrique et composé de 13,2 ml d'une solution aqueuse d'acide nitrique à 49 %, 106 mg de métavanadate d'ammonium et 50 mg de nitrite de sodium. Le milieu réactionnel est maintenu alors 6 heures à 75°C, puis 2 heures à 90°C.

En fin de réaction, le milieu réactionnel est refroidi.

Le pH est alors ramené à 2,4 par ajout d'une solution aqueuse de soude 36 %.

Le mélange est alors amené à 500 ml et dosé par électrophorèse capillaire.

Les résultats obtenus sont les suivants :

- acide pélargonique : 244 mg
- acide caprylique : 45 mg
- acide heptanoïque : 13 mg
- acide azélaïque : 212 mg
- acide subérique : 24 mg
- acide pimélique : 18 mg

La sélectivité en acide azélaïque est de 82 %.

La sélectivité en acide pélargonique est de 79 %.

EXEMPLE 3:

Dans cet exemple, on effectue la coupure oxydante de l'acide oléique en présence d'acide formique.

Dans un ballon tricol de 50 ml muni d'une agitation mécanique, d'un thermomètre, et d'un dispositif de reflux, on charge 867 mg d'acide oléique (3,07 mmol) et 5 g d'acide formique.

On ajoute alors 797 mg d'une solution aqueuse d'eau oxygénée à 30 % (7 mmol) et l'on chauffe le milieu réactionnel 2 heures à 40°C.

On ajoute ensuite directement sur le milieu réactionnel un mélange contenant 39 g d'une solution aqueuse d'acide

nitrique à 49 %, 222 mg de métavanadate d'ammonium, et 100 mg de nitrite de sodium. Le milieu réactionnel est alors maintenu 6 heures à 60°C.

Le milieu réactionnel est alors refroidi, neutralisé à pH = 2,3 avec une solution aqueuse de soude 36 %, dilué à 1 litre et dosé par électrophorèse capillaire.

Les résultats obtenus sont les suivants :

- acide pélargonique : 247 mg
- acide caprylique : 15 mg
- acide heptanoïque : 11 mg
- acide azélaïque : 284 mg
- acide subérique : 24 mg
- acide pimélique : 4 mg

La sélectivité en acide pélargonique est de 90 %.

La sélectivité en acide azélaïque est de 90 %.

EXEMPLE 4:

Dans cet exemple on effectue la coupure oxydante de l'acide érucique en présence d'acide formique.

Dans un réacteur agité muni d'un réfrigérant et d'une sonde de température on charge 1,21 g d'acide érucique (3,57 mmol), 0,36 g d'acide formique et 0,8 g d'eau oxygénée à 30 % (7,05 mmol). On chauffe alors le milieu réactionnel 7h à 80°C. On ajoute ensuite directement sur le milieu réactionnel 13,4 g d'une solution aqueuse d'acide nitrique à 55 %, 70 mg de nitrite de sodium et 133 mg de métavanadate d'ammonium. Le milieu réactionnel est alors maintenu 5h3 à 61°C. Le milieu réactionnel est alors refroidi, neutralisé à pH=2,3 avec une solution aqueuse de soude à 36 %. Le milieu réactionnel est alors estérifié et dosé par chromatographie en phase gaz.

Les résultats obtenus sont les suivants :

- diméthylbrassylate : 465 mg
- diméthyldodécanedioate : 109 mg
- diméthylsubérate : 1 mg
- pélargonate de méthyle : 291 mg
- caprylate de méthyle : 52 mg
- heptanoate de méthyle : 2 mg

La sélectivité en acide brassylique est de 80 %.

La sélectivité en acide pélargonique est de 83 %.

EXEMPLE 5 :

Dans cet exemple on effectue la coupure oxydante de l'acide pétrosélénique en présence d'acide formique.

Dans un réacteur agité muni d'un réfrigérant et d'une sonde de température on charge 0,99 g d'acide érucique (3,51 mmol), 0,37 g d'acide formique et 0,8 g d'eau oxygénée à 30 % (7,05 mmol). On chauffe alors le milieu réactionnel 7h à 80°C. On ajoute ensuite directement sur le milieu réactionnel 13,4 g d'une solution aqueuse d'acide nitrique à 55 %, 70 mg de nitrite de sodium et 133 mg de métavanadate d'ammonium. Le milieu réactionnel est alors maintenu 5h3 à 61°C. Le milieu réactionnel est alors refroidi, neutralisé à pH=2,3 avec une solution aqueuse de soude à 36 %. Le milieu réactionnel est alors estérifié et dosé par chromatographie en phase gaz.

Les résultats obtenus sont les suivants :

- diméthyladipate : 287 mg
- diméthylglutarate : 40 mg
- laurate de méthyle : 400 mg
- undécanoate de méthyle : 65 mg
- décanoate de méthyle : 4 mg

La sélectivité en acide adipique est de 80 %.

La sélectivité en acide laurique est de 78 %.

EXEMPLE 6:

Dans cet exemple on effectue la coupure oxydante de l'oléate de méthyle en présence d'acide formique.

Dans un réacteur agité muni d'un réfrigérant et d'une sonde de température on charge 1,049 g d'oléate de méthyle (3,54 mmol), 0,36 g d'acide formique et 0,8 g d'eau oxygénée à 30 % (7,05 mmol). On chauffe alors le milieu réactionnel 7h à 80°C.

On ajoute ensuite directement sur le milieu réactionnel 13,4 g d'une solution aqueuse d'acide nitrique à 55 %, 70 mg de nitrite de sodium et 133 mg de métavanadate d'ammonium. Le milieu réactionnel est alors maintenu 5h30 à 61°C. Le milieu réactionnel est alors refroidi, neutralisé à pH=2,3 avec une solution aqueuse de soude à 36 %. Le milieu réactionnel est alors estérifié et dosé par chromatographie en phase gaz.

Les résultats obtenus sont les suivants :

- diméthylazélate : 495 mg
- diméthylsubérate : 51 mg
- diméthylpiméllate : 4 mg
- pélargonate de méthyle : 384 mg
- caprylate de méthyle : 39 mg
- heptanoate de méthyle : 5 mg

La sélectivité en acide azélaique est de 89 %.

La sélectivité en acide pélargonique est de 89 %.

EXEMPLE 7:

Dans cet exemple on effectue la coupure oxydante de l'acide oléique en présence d'eau oxygénée seule.

Dans un réacteur agité muni d'un réfrigérant et d'une sonde de température on charge 1,01 g d'acide oléique (3,59 mmol) et 0,8 g d'eau oxygénée à 30 % (7,05 mmol). On chauffe alors le milieu réactionnel 7h à 80°C.

On ajoute ensuite directement sur le milieu réactionnel 13,4 g d'une solution aqueuse d'acide nitrique à 55 %, 70 mg de nitrite de sodium et 133 mg de métavanadate d'ammonium. Le milieu réactionnel est alors maintenu 5h à 61°C.

Le milieu réactionnel est alors refroidi, neutralisé à pH=2,3 avec une solution aqueuse de soude à 36 %. Le milieu réactionnel est alors estérifié et dosé par chromatographie en phase gaz.

Les résultats obtenus sont les suivants :

- diméthylazélate : 76 mg
- diméthylsubérate : 45 mg
- diméthylpiméllate : 8 mg
- diméthyladipate : 24 mg
- pélargonate de méthyle : 119 mg
- caprylate de méthyle : 41 mg
- heptanoate de méthyle : 6 mg

La sélectivité en acide azélaique est de 50 %.

La sélectivité en acide pélargonique est de 68 %.

EXEMPLE 8:

Dans cet exemple on effectue la coupure oxydante de l'acide oléique en présence d'eau oxygénée et d'acide sulfurique.

Dans un réacteur agité muni d'un réfrigérant et d'une sonde de température on charge 1,01 g d'acide oléique (3,59 mmol), 0,12 g d'acide sulfurique à 98 % et 0,8 g d'eau oxygénée à 30 % (7,05 mmol). On chauffe alors le milieu réactionnel 7h à 80°C.

On ajoute ensuite directement sur le milieu réactionnel 13,4 g d'une solution aqueuse d'acide nitrique à 55 %, 70 mg de nitrite de sodium et 133 mg de métavanadate d'ammonium. Le milieu réactionnel est alors maintenu 5h à 61°C.

Le milieu réactionnel est alors refroidi, neutralisé à pH=2,3 avec une solution aqueuse de soude à 36 %. Le milieu réactionnel est alors estérifié et dosé par chromatographie en phase gaz.

Les résultats obtenus sont les suivants :

- diméthylazélate : 316 mg

. diméthylsubérate : 136 mg
. diméthylpiméllate : 13 mg
. diméthyladipate : 6 mg
. pélargonate de méthyle : 238 mg
. caprylate de méthyle : 91 mg
. heptanoate de méthyle : 7 mg

La sélectivité en acide azélaique est de 71 %.
La sélectivité en acide pélargonique est de 60 %.

EXEMPLE 9 :

Dans cet exemple on effectue la coupure oxydante de l'acide oléique en présence d'acide tungstique.

Dans un réacteur agité muni d'un réfrigérant et d'une sonde de température on charge 1,0 g d'acide oléique (3,54 mmol), 0,206 g d'acide tungstique et 0,8 g d'eau oxygénée à 30 % (7,05 mmol). On chauffe alors le milieu réactionnel 5h à 80°C.

On ajoute ensuite directement sur le milieu réactionnel 13,4 g d'une solution aqueuse d'acide nitrique à 55 %, 70 mg de nitrite de sodium et 133 mg de métavanadate d'ammonium. Le milieu réactionnel est alors maintenu 6h à 61°C.

Le milieu réactionnel est alors refroidi, neutralisé à pH=2,3 avec une solution aqueuse de soude à 36 %. Le milieu réactionnel est alors estérifié et dosé par chromatographie en phase gaz.

Les résultats obtenus sont les suivants :

. diméthylazélate : 544 mg
. diméthylsubérate : 82 mg
. diméthylpiméllate : 8 mg
. diméthyladipate : 5 mg
. pélargonate de méthyle : 416 mg
. caprylate de méthyle : 58 mg
. heptanoate de méthyle : 4 mg

La sélectivité en acide azélaique est de 84 %.
La sélectivité en acide pélargonique est de 86 %.

## Revendications

1. Procédé de préparation d'acides mono- et dicarboxyliques, à partir d'acides gras insaturés et/ou leurs dérivés caractérisé par le fait qu'il comporte les étapes suivantes :

   - une étape qui consiste à oxyder un acide gras insaturé sous forme d'acide et/ou d'ester par le peroxyde d'hydrogène, en présence d'un oxyde métallique ou d'un acide carboxylique éventuellement associé à un catalyseur à base de ruthénium,
   - une étape qui consiste à faire réagir le milieu réactionnel issu de l'étape précédente, avec de l'acide nitrique en présence d'un catalyseur à base de vanadium, éventuellement associé à un co-catalyseur,
   - puis une étape de récupération des acides mono- et dicarboxyliques.

2. Procédé selon la revendication 1 caractérisé par le fait que le substrat de départ comprend au moins un acide gras présentant au moins une double liaison.

3. Procédé selon la revendication 2 caractérisé par le fait que l'acide gras insaturé répond à la formule (I) :

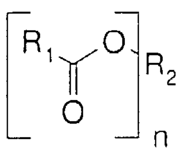

(I)

dans laquelle :

- n est un nombre égal à 1, 2 ou 3,
- $R_1$ représente un radical alcényle ou alcadiényle, linéaire ou ramifié ayant de 4 à 40 atomes de carbone,
- $R_2$ peut prendre les significations suivantes :

  . si n = 1, $R_2$ représente un atome d'hydrogène, ayant de préférence de 1 à 6 atomes de carbone, éventuellement porteur d'un ou plusieurs groupements hydroxyle,
  . si n = 2, $R_2$ représente un radical alkylène ou alcénylène, ayant de préférence de 1 à 6 atomes de carbone, éventuellement porteur d'un ou plusieurs groupements hydroxyle,
  . si n = 3, $R_2$ représente un radical alkyle ayant 3 atomes de carbone.

**4.** Procédé selon l'une des revendications 1 à 3 caractérisé par le fait que l'acide gras insaturé répond à la formule (I) dans laquelle $R_1$ représente un radical alcényle ayant de 6 à 22 atomes de carbone et présentant de 1 à 5 doubles liaisons, de préférence 1 à 3 et $R_2$ représente un atome d'hydrogène ou un radical alkyle ayant de 1 à 4 atomes de carbone porteur éventuellement de 1 à 2 groupes hydroxyle, pour n = 1 ; un radical diéthylène ou propylène éventuellement porteur d'un groupe hydroxyle pour n = 2 ; un radical propanetriyle-1,2,3 pour n = 3.

**5.** Procédé selon l'une des revendications 1 à 4 caractérisé par le fait que le substrat de départ est choisi parmi :

- les acides gras insaturés présentant une seule double liaison tel que l'acide lindérique, l'acide myristoléique, l'acide palmitoléique, l'acide oléique, l'acide pétrosélénique, l'acide doeglique, l'acide gadoléique, l'acide érucique ; les acides gras insaturés présentant deux doubles liaisons tels que l'acide linoléique ; les acides gras insaturés présentant 3 doubles liaisons tels que l'acide linolénique ; les acides gras insaturés présentant plus de 4 doubles liaisons tels que l'acide isanique, l'acide stéarodonique, l'acide arachidonique, l'acide chypanodonique ; les acides gras insaturés porteurs de groupe hydroxyle tel que l'acide ricinoléique ainsi que leurs mélanges,
- les huiles et les graisses d'origine animale, de préférence, l'huile de cachalot, l'huile de dauphin, l'huile de baleine, l'huile de phoque, l'huile de sardine, l'huile de hareng, l'huile de squale, l'huile de foie de morue ; l'huile de pied de boeuf, les graisses de boeuf, de porc, de cheval, de mouton (suifs),
- les huiles d'origine végétale, de préférence, l'huile de colza, l'huile de tournesol, l'huile d'arachide, l'huile d'olive, l'huile de noix, l'huile de maïs, l'huile de soja, l'huile de lin, l'huile de chanvre, l'huile de pépins de raisin, l'huile de coprah, l'huile de palme, l'huile de graines de coton, l'huile de babassu, l'huile de jojoba, l'huile de sésame, l'huile de ricin,
- les esters correspondants aux acides gras précités, en particulier les esters méthylique, éthylique et propylique et plus particulièrement les produits de méthanolyse notamment des huiles, et plus préférentiellement des huiles de colza.

**6.** Procédé selon l'une des revendications 1 à 5 caractérisé par le fait que le substrat de départ est l'acide palmitoléique, l'acide oléique, l'acide pétrosélénique, l'acide érucique, l'acide linoléique, l'acide linolénique, l'acide ricinoléique ; l'huile de colza, l'huile de tournesol, l'huile de soja, l'huile de lin, l'huile de ricin, de préférence, l'acide oléique, l'huile de colza, l'huile de tournesol et l'huile de soja.

**7.** Procédé selon l'une des revendications 1 à 6 caractérisé par le fait que la quantité de peroxyde d'hydrogène mise en oeuvre est une quantité égale à la stoechiométrie ou un léger excès allant de 0 à 50 %, de préférence, compris entre 1 et 20 %.

**8.** Procédé selon l'une des revendications 1 à 7 caractérisé par le fait que le peroxyde d'hydrogène est associé à un acide carboxylique.

**9.** Procédé selon la revendication 8 caractérisé par le fait que l'acide carboxylique répond à la formule générale (II) :

$$R_3 - COOH \qquad (II)$$

dans ladite formule (II), $R_3$ représente un radical hydrocarboné ayant de 1 à 22 atomes de carbone qui peut être un radical aliphatique acyclique saturé, linéaire ou ramifié, un radical cycloaliphatique saturé, monocyclique ou

polycyclique.

10. Procédé selon l'une des revendications 8 et 9 caractérisé par le fait que l'acide carboxylique est un acide carboxylique aliphatique saturé de préférence, l'acide formique ou l'acide acétique ou un acide formé au cours de la réaction, de préférence, l'acide pélargonique, l'acide azélaïque et l'acide stéarique.

11. Procédé selon l'une des revendications 8 à 10 caractérisé par le fait que la quantité d'acide carboxylique mise en oeuvre est telle que le rapport molaire entre le peroxyde d'hydrogène et l'acide carboxylique varie entre 10 et 500 %, de préférence entre 10 et 40 %.

12. Procédé selon l'une des revendications 8 à 11 caractérisé par le fait que l'on ajoute un catalyseur à base de ruthénium.

13. Procédé selon l'une des revendications 8 à 12 caractérisé par le fait que le catalyseur à base de ruthénium est choisi parmi : le chlorure de ruthénium III, le chlorure de ruthénium IV, le pentafluorure de ruthénium, l'oxyde de ruthénium II, l'oxyde de ruthénium IV, l'oxychlorure de ruthénium ammoniaqué $Ru_2(OH)_2Cl_4$, $7NH_3$, $5H_2O$, l'acétate de ruthénium.

14. Procédé selon l'une des revendications 12 et 13 caractérisé par le fait que la quantité de catalyseur à base de ruthénium mise en oeuvre, exprimée par le rapport pondéral entre le peroxyde d'hydrogène et le catalyseur est comprise entre 1 et 35 %, de préférence entre 3 et 10 %.

15. Procédé selon l'une des revendications 1 à 7 caractérisé par le fait que le peroxyde d'hydrogène est associé à un catalyseur à base d'un métal du groupe VIa de la classification périodique des éléments, de préférence, un catalyseur à base de tungstène et/ou de molybdène.

16. Procédé selon l'une des revendications 1 à 7 et 15 caractérisé par le fait que le catalyseur est choisi de préférence parmi : l'acide tungstique, l'acide phosphotungstique, l'acide molybdique, l'acide phosphomolybdique ou leurs précurseurs.

17. Procédé selon l'une des revendications 1 à 7, 15 et 16 caractérisé par le fait que la quantité de catalyseur à base de tungstène et/ou molybdène mise en oeuvre, exprimée par le rapport pondéral entre le peroxyde d'hydrogène et le catalyseur est comprise entre 1 et 35 %, de préférence entre 3 et 10 %.

18. Procédé selon l'une des revendications 16 et 17 caractérisé par le fait que l'on ajoute un acide fort, de préférence, l'acide nitrique, l'acide sulfurique ou l'acide phosphorique.

19. Procédé selon l'une des revendications 1 à 18 caractérisé par le fait que la température de la première étape d'oxydation est comprise entre 30 et 100°C, de préférence entre 60°C et 100°C.

20. Procédé selon l'une des revendications 1 à 19 caractérisé par le fait que la quantité d'acide nitrique mise en jeu dans la deuxième étape représente de 2 à 50 fois le poids du substrat de départ et plus préférentiellement de 4 à 10 fois.

21. Procédé selon la revendication 20 caractérisé par le fait que l'on ajoute une source de $NO^+$ choisi parmi : le dioxyde d'azote $NO_2$, l'anhydride azoteux $N_2O_3$, le peroxyde d'azote $N_2O_4$, l'oxyde d'azote NO, l'acide nitreux, un sulfate de nitrosyle ou nitrose, un sel nitreux, de préférence un sel de métal alcalin, et encore plus préférentiellement, de sodium.

22. Procédé selon l'une des revendications 20 et 21 caractérisé par le fait que le catalyseur à base de vanadium est choisi parmi :

- les halogénures de vanadium tels que le tri- tétra- ou pentafluorure de vanadium, le di- tri- ou tétrachlorure de vanadium, le tribromure de vanadium,
- les oxydes de vanadium tels que l'oxyde de vanadium, le dioxyde de vanadium, le sesquioxyde de vanadium, le pentaoxyde de vanadium,
- les oxyhalogénures de vanadium en particulier les oxy di- ou trifluorure de vanadium, les oxy mono- di- ou trichlorure de vanadium, les oxy mono- di- ou tribromure de vanadium,

- le sulfate de vanadium,
- le sulfate de vanadyle,
- les vanadates de métaux alcalins ou d'ammonium sous forme ortho- méta- ou pyro,
- l'acétylacétonate de vanadyle.

**23.** Procédé selon l'une des revendications 20 à 22 caractérisé par le fait que la quantité de catalyseur à base de vanadium mise en oeuvre, exprimée par le rapport pondéral entre le catalyseur exprimée en $HVO_3$ et l'acide nitrique est comprise de préférence, entre 0,001 et 1 %, de préférence entre 0,02 et 0,5 %.

**24.** Procédé selon l'une des revendications 20 à 23 caractérisé par le fait que l'on ajoute un co-catalyseur à base d'un métal du groupe VIIa et VIII de la classification périodique, de préférence, un catalyseur à base de manganèse, fer, nickel, ruthénium et/ou cobalt.

**25.** Procédé selon l'une des revendications 20 à 24 caractérisé par le fait que l'on effectue l'étape d'oxydation nitrique, en présence d'oxygène ou un gaz en contenant.

**26.** Procédé selon la revendication 25 caractérisé par le fait que la quantité d'oxygène représente de 1 à 2 fois la quantité stoechiométrique lorsque le substrat de départ est un acide gras insaturé et de 5 à 8 fois la quantité stoechiométrique quand la matière première est une huile ou une graisse.

**27.** Procédé selon l'une des revendications 20 à 26 caractérisé par le fait que la température de la réaction dans la deuxième étape est choisie de préférence, entre 40°C et 100°C, et plus préférentiellement entre 60 et 90°C.

**28.** Procédé selon l'une des revendications 1 à 27 caractérisé par le fait que l'on obtient un milieu bi-phasique avec une phase aqueuse comprenant les acides gras saturés dicarboxyliques, l'acide nitrique, les catalyseurs et co-catalyseurs et une phase organique comprenant essentiellement les acides gras saturés monocarboxyliques formés au cours de la réaction et éventuellement ceux présents au départ ainsi que les acides gras insaturés qui n'ont pas réagi et que l'on sépare les acides gras saturés mono- et dicarboxyliques obtenus.

**Patentansprüche**

**1.** Verfahren zur Herstellung von Mono- und Dicarbonsäuren ausgehend von ungesättigten Fettsäuren und/oder deren Derivaten, dadurch gekennzeichnet, daß es die folgenden Stufen umfaßt:

- eine Stufe, welche darin besteht, eine ungesättigte Fettsäure in Säure- und/oder Esterform durch Wasserstoffperoxid in Gegenwart eines Metalloxides oder einer Carbonsaure, gegebenenfalls verbunden mit einem Katalysator auf Basis von Ruthenium, zu oxidieren,
- eine Stufe, welche darin besteht, das in der vorherigen Stufe erhaltende Reaktionsmedium mit Salpetersäure in Gegenwart eines Katalysators auf Basis von Vanadium, gegebenenfalls verbunden mit einem Co-Katalysator reagieren zu lassen,
- dann eine Stufe der Gewinnung der Mono- und Dicarbonsäuren.

**2.** Verfahren nach Anspruch 1, dadurch gekennzeichnet, daß das Ausgangsmaterial wenigstens eine Fettsäure umfaßt, welche wenigstens eine Doppelbindung aufweist.

**3.** Verfahren nach Anspruch 2, dadurch gekennzeichnet, daß die ungesättigte Fettsäure der Formel (I) entspricht:

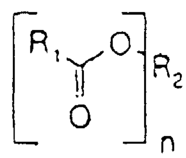

(I)

worin:

- n eine Zahl gleich 1, 2 oder 3 ist,
- $R_1$ ein linearer oder verzweigter Alkenyl- oder Alkadienylrest mit 4 bis 40 Kohlenstoffatome ist,
- $R_2$ die folgenden Bedeutungen annehmen kann:

  - wenn n = 1, ist $R_2$ ein Wasserstoffatom mit vorzugsweise 1 bis 4 Kohlenstoffatomen, die gegebenenfalls Träger einer oder mehrerer Hydroxylgruppen sind;
  - wenn n = 2, ist $R_2$ ein Alkenyl- oder Alkenylenrest mit vorzugsweise 1 bis 6 Kohlenstoffatomen und trägt gegebenenfalls ein oder mehrere Hydroxylgruppen,
  - wenn n = 3, ist $R_3$ ein Alkkylrest mit 3 Kohlenstoffatomen.

**4.** Verfahren nach einem der Ansprüche 1 bis 3, dadurch gekennzeichnet, daß die ungesättigte Fettsäure der Formel (I) entspricht, worin $R_1$ einen Alkenylrest mit 6 bis 22 Kohlenstoffatomen und 1 bis 5, vorzugsweise 1 bis 3 Doppelbindungen darstellt und $R_2$ ein Wasserstoffatom oder einen Alkylrest mit 1 bis 4 Kohlenstoffatomen, welche gegebenenfalls 1 bis 2 Hydroxylgruppen aufweisen, für n = 1, einen Diethylen- oder Propylenrest, welcher gegebenenfalls eine Hydroxylgruppe trägt, für n = 2, einen 1,2,3-Propantriylrest, für n = 3, darstellt.

**5.** Verfahren nach einem der Ansprüche 1 bis 4, dadurch gekennzeichnet, daß das Ausgangsmaterial ausgewählt ist aus;

- den ungesättigten Fettsäuren mit einer einzigen Doppelbindung, wie Lindersäure, Myristolsäure, palmiloleinsäure, Oleinsäure, Petroselinsäure, Doegleinsäure, Gadoleinsäure, Erucasäure; den ungesättigten Fettsäuren mit zwei Doppelbindungen, wie Linolsäure; den ungesättigten Fettsäuren mit drei Doppelbindungen, wie Linolensäure; den ungesättigten Fettsäuren mit mehr als vier Doppelbindungen, wie Isansäure, Stearodonsäure, Arachidonsäure, Chypanodonsäure; den ungesättigten Fettsäuren mit Hydroxylgruppe, wie Ricinolsäure sowie deren Mischungen;
- den Ölen und Fetten tierischen Ursprungs, vorzugsweise Pottwalöl, Delphinöl, Walöl, Seehundöl, Sardinenöl, Heringsöl, Haiöl, Lebertran; Rinderfußöl, den Fetten von Rind, Schwein, Pferd, Schaf (Talg);
- den Ölen pflanzlichen Ursprungs, vorzugsweise Rüböl, Sonnenblumenöl, Erdnußöl, Olivenöl, Nußöl, Maisöl, Sojaöl, Leinöl, Hanföl, Traubenkernöl, Kopraöl, Palmöl, Baumwollsamenöl, Babassuöl, Jojobaöl, Seesamöl, Ricinusöl;
- den den vorgenannten Fettsäuren entsprechenden Estern, insbesondere die Methyl-, Ethyl- und Propylester und ganz besonders die Produkte der Methylierung insbesondere von Ölen, und am bevorzugtesten von Rüböl.

**6.** Verfahren nach einem der Ansprüche 1 bis 5, dadurch gekennzeichnet, daß das Ausgangsmaterial Palmitoleinsäure, Oleinsäure, Petroselinsäure, Erucasäure, Linolsäure, Linolensäure, Ricinolsäure; Rüböl, Sonnenblumenöl, Sojaöl, Leinöl , Ricinusöl, vorzugsweise Oleinsäure, Ruböl, , Sonnenblumenöl und Sojaöl ist.

**7.** Verfahren nach einem der Ansprüche 1 bis 6, dadurch gekennzeichnet, daß die Menge an eingesetztem Wasserstoffperoxid eine stöchiometrische oder leicht überstöchiometrische Menge von 0 bis 50 %, vorzugsweise zwischen 1 und 20 %, ist.

**8.** Verfahren nach einem der Ansprüche 1 bis 7, dadurch gekennzeichnet, daß das Wasserstoffperoxid mit einer Carbonsäure verbunden ist.

**9.** Verfahren nach Anspruch 8, dadurch gekennzeichnet, daß die Carbonsäure der allgemeinen Formel (II)

$$R_3\text{-COOH}$$

- entspricht, worin $R_3$ ein Kohlenwasserstoffrest mit 1 bis 22 Kohlenstoffatomen ist, welcher ein linearer oder verzweigter, gesättigter aliphatischer, acyclischer Rest, ein monocyclischer oder polycyclischer gesättigter cycloaliphatischer Rest sein kann.

**10.** Verfahren nach einem der Ansprüche 8 und 9, dadurch gekennzeichnet, daß die Carbonsäure eine gesättigte aliphatische Carbonsäure, vorzugsweise Ameisensäure oder Essigsäure oder eine im Verlauf der Reakticn erzeugte Saure, vorzugsweise Pelargonsäure, Azelainsäure und Stearinsäure ist.

**11.** Verfahren nach einem der Ansprüche 8 bis 10, dadurch gekennzeichnet, daß die Menge an eingesetzter Carbonsäure derart ist, daß das Molverhältnis zwischen dem Wasserstoffperoxid und der Carbonsaure zwischen 10 und 500 %, vorzugsweise zwischen 10 und 40 %, variiert.

**12.** Verfahren nach einem der Ansprüche 8 bis 11, dadurch gekennzeichnet, daß man einen Katalysator auf Basis von Ruthenium zusetzt.

**13.** Verfahren nach einem der Ansprüche 8 bis 12, dadurch gekennzeichnet, daß der Katalysator auf Basis von Ruthenium aus Ruthenium(III)chlorid, Ruthenium(IV)chlorid, Ruthenium-pentafluorid, Ruthenium(II)oxid, Ruthenium (IV)oxid, amoniakalischem Rutheniumoxychlorid $Ru_2(OH)_2Cl_4$, $7NH_3$, $5H_2O$, Rutheniumacetat ist.

**14.** Verfahren nach einem der Ansprüche 12 und 13, dadurch gekennzeichnet, daß die eingesetzte Menge an Katalysator auf Basis von Ruthenium, ausgedrückt im Gewichtsverhältnis zwischen Wasserstoffperoxid und dem Katalysator, zwischen 1 und 35 %, vorzugsweise zwischen 3 und 10 %, enthalten ist.

**15.** Verfahren nach einem der Ansprüche 1 bis 7, dadurch gekennzeichnet, daß das Wasserstoffperoxid mit einem Katalysator auf Basis eines Metalles der Gruppe VIa des Periodensystems, vorzugsweise ein Katalysator auf Basis von Wolfram und/oder Molybdän ist.

**16.** Verfahren nach einem der Ansprüche 1 bis 7 und 15, dadurch gekennzeichnet, daß der Katalysator vorzugsweise aus Wolframsäure, Phosphowolframsäure, molybdänsäure, Phosphomolybdänsäure und deren Vorläufer ausgewählt ist.

**17.** Verfahren nach einem der Ansprüche 1 bis 7, 15 und 16, dadurch gekennzeichnet, daß die eingesetzte Menge an Katalysator auf Basis von Wolfram und/oder Molybdän, ausgedrückt durch das Gewichtsverhältnis zwischen Wasserstoffperoxid und dem Katalysator, zwischen 1 und 35 %, vorzugsweise zwischen 3 und 10 %, enthalten ist.

**18.** Verfahren nach einem der Ansprüche 16 und 17, dadurch gekennzeichnet, daß man eine starke Säure, vorzugsweise Salpetersäure, Schwefelsäure oder Phosphorsäure zusetzt.

**19.** Verfahren nach einem der Ansprüche 1 bis 18, dadurch gekennzeichnet, daß die Temperatur der ersten Stufe der Oxidation zwischen 30 und 100°C, vorzugsweise zwischen 60°C und 100°C, enthalten ist.

**20.** Verfahren nach einem der Ansprüche 1 bis 19, dadurch gekennzeichnet, daß die in der zweiten Stufe eingesetzte Menge an Salpetersäure das 2- bis 50-fache und am bevorzugtesten das 4- bis 10-fache des Gewichtes des Ausgangssutstrates beträgt.

**21.** Verfahren nach Anspruch 20, dadurch gekennzeichnet, daß man eine $NO^+$-Quelle zusetzt, ausgewählt aus Stickstoffdioxid $NO_2$, Salpetrigsäureanhydrid $N_2O_3$, Stickstoffperoxid $N_2O_4$, Stickoxid NO, Salpetrigsäure, einem Nitrosyl- oder Nitrososulfat, einem salpetrigsauren Salz, vorzugsweise einem Alkalimetall-, und am bevorzugtesten Natriumsalz.

**22.** Verfahren nach einem der Ansprüche 20 und 21, dadurch gekennzeichnet, daß der Katalysator auf Basis von Vanadium ausgewählt ist aus:

- den Vanadiumhalogeniden, wie dem Tri-, Tetra- oder Pentafluoridvanadium, dem Tri- oder Tetrachlorid von Vanadium, dem Tribromid von Vanadium,
- den Vanadiumoxiden, wie Vanadiumoxid, Vanadiumdioxid, Vanadiumsesquioxid, Vanadiumpentoxid,
- den Vanadiumoxihalogeniden, insbesondere dem Oxidi- oder Oxitrifluorid von Vanadium, den Oxymono-, -di- oder -trichloriden von Vanadium, den Oximono-, -di- oder -tribromiden von Vanadium,
- dem Vanadiumsulfat,
- dem Vanadylsulfat,
- den Alkalimetall- oder Ammoniumvanadaten in Form von Ortho-, Meta- oder Pyrovanadaten,
- dem Vanadylacetylacetonat.

**23.** Verfahren nach einem der Ansprüche 20 bis 22, dadurch gekennzeichnet, daß die eingesetzte Menge an Katalysator auf Basis von Vanadium, ausgedrückt durch das Gewichtsverhältnis zwischen dem Katalysator, ausgedrückt in $HVO_3$ und Salpetersäure, vorzugsweise zwischen 0,001 und 1 %, bevorzugt zwischen 0,02 und 0,5 %, enthalten

ist.

**24.** Verfahren nach einem der Ansprüche 20 bis 23, dadurch gekennzeichnet, daß man einen Co-Katalysator auf Basis eines Metalles der Gruppe VII und VIII des Periodensystems, vorzugsweise einen Katalysator auf Basis von Mangan, Eisen, Nickel, Ruthenium und/oder Cobalt zusetzt.

**25.** Verfahren nach einem der Ansprüche 20 bis 24, dadurch gekennzeichnet, daß man die Stufe der Nitroxidation in Gegenwart von Sauerstoff oder einem sauerstoffhaltigen Gas durchführt.

**26.** Verfahren nach Anspruch 25, dadurch gekennzeichnet, daß die Menge an Sauerstoff das 1- bis 2-fache der stöchiometrischen Menge darstellt, wenn das Ausgangsmaterial eine ungesättigte Fettsäure ist, und das 5- bis 3-fach der stöchiometrischen Menge, wenn das Ausgangsmaterial ein Öl oder ein Fett ist.

**27.** Verfahren nach einem der Ansprüche 20 bis 25, dadurch gekennzeichnet, daß die Temperatur der Umsetzung in der zweiten Stufe vorzugsweise zwischen 40 und 100°C und bevorzugter zwischen 60 und 90°C ausgewählt wird.

**28.** Verfahren nach einem der Ansprüche 1 bis 27, dadurch gekennzeichnet, daß man ein zweiphasiges Milieu mit einer wäßrigen Phase, welche die gesättigte dicarbonische Fettsäure, Salpetersäure, die Katalysatoren und Co-Katalysatoren enthält und einer organischen Phase, welche im wesentlichen die im Verlauf der Umsetzung erzeugten monocarbonischen, gesättigten Fettsäuren und gegebenenfalls jene des Ausgangsmateriales sowie die ungesättigte Fettsäuren, welche nicht reagiert haben, umfaßt, erhält und daß man die erhaltenen mono- und dicarbonischen gesättigten Fettsäuren abtrennt.

## Claims

**1.** A process for preparing mono- and dicarboxylic acids from unsaturated fatty acids and/or their derivatives, characterized in that it comprises the following steps:

- a step consisting of oxidising an unsaturated fatty acid in the form of the acid and/or ester using hydrogen peroxide, in the presence of a metal oxide or a carboxylic acid optionally associated with a ruthenium based catalyst;
- a step consisting of reacting the reaction medium from the preceding step with nitric acid in the presence of a vanadium based catalyst, optionally associated with a co-catalyst;
- then a step for recovering the mono- and dicarboxylic acids.

**2.** A process according to claim 1, characterized in that the starting substrate comprises at least one fatty acid containing at least one double bond.

**3.** A process according to claim 2, characterized in that the unsaturated fatty acid has formula (I):

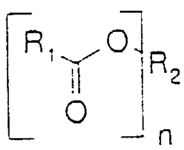

(I)

where:

- n is a number equal to 1, 2 or 3;
- $R_1$ represents a linear or branched alkenyl or alkadienyl radical containing 4 to 40 carbon atoms;
- $R_2$ can take the following meanings:
  - when n = 1, $R_2$ represents a hydrogen atom, preferably containing 1 to 6 carbon atoms, optionally carrying one or more hydroxyl groups;
  - when n = 2, $R_2$ represents an alkylene or alkenylene radical, preferably containing 1 to 6 carbon atoms,

optionally carrying one or more hydroxyl groups;
- when n = 3, $R_2$ represents an alkyl radical containing 3 carbon atoms.

**4.** A process according to any one of claims 1 to 3, characterized in that the unsaturated fatty acid has formula (I) where $R_1$ represents an alkenyl radical containing 6 to 22 carbon atoms and containing 1 to 5 double bonds, preferably 1 to 3 and $R_2$ represents a hydrogen atom or an alkyl radical containing 1 to 4 carbon atoms optionally carrying 1 or 2 hydroxyl groups, for n = 1; a diethylene or propylene radical optionally carrying a hydroxyl group for n = 2; or a propanetri-1,2,3-yl radical for n = 3.

**5.** A process according to any one of claims 1 to 4, characterized in that the starting substrate is selected from:

- unsaturated fatty acids containing a single double bond such as linderic acid, myristoleic acid, palmitoleic acid, oleic acid, petroselenic acid, doeglic acid, gadoleic acid, erucic acid; unsaturated fatty acids containing two double bonds such as linoleic acid; unsaturated fatty acids containing 3 double bonds such as linolenic acid; unsaturated fatty acids containing more than 4 double bonds such as isanic acid, stearodonic acid, arachidonic acid, chypanodonic acid; and unsaturated fatty acids carrying a hydroxyl group such as ricinoleic acid, and mixtures thereof;
- animal oils and fats, preferably sperm whale oil, dolphin oil, whale oil, seal oil, sardine oil, herring oil, shark oil, cod liver oil, neatsfoot oil, beef fat, pork fat, horse fat, and sheep fat (tallow);
- vegetable oils, preferably rapeseed oil, sunflower oil, peanut oil, olive oil, walnut oil, corn oil, soya oil, linseed oil, hempseed oil, grapeseed oil, coprah oil, palm oil, cottonseed oil, babassu oil, jojoba oil, sesame oil, and castor oil;
- the esters corresponding to the above fatty acids, in particular the methyl, ethyl and propyl esters, more particularly methanolysis products in particular of the oils, more preferably of rapeseed oils.

**6.** A process according to any one of claims 1 to 5, characterized in that the starting substrate is palmitoleic acid, oleic acid, petroselenic acid, erucic acid, linoleic acid, linolenic acid, ricinoleic acid; rapeseed oil, sunflower oil, soya oil, linseed oil, or castor oil, preferably oleic oil, rapeseed oil. sunflower oil or soya oil.

**7.** A process according to any one of claims 1 to 6, characterized in that the quantity of hydrogen peroxide used is a quantity equal to the stoichiometric quantity or a slight excess of 0 to 50%, preferably in the range 1% to 20%.

**8.** A process according to any one of claims 1 to 7, characterized in that the hydrogen peroxide is associated with a carboxylic acid.

**9.** A process according to claim 8, characterized in that the carboxylic acid has general formula (II):

$$R_3\text{-COOH} \qquad \text{(II)}$$

in which formula (II) $R_3$ represents a hydrocarbon radical containing 1 to 22 carbon atoms, which may be a linear or branched saturated aliphatic acyclic radical, or a monocyclic or polycyclic saturated cycloaliphatic radical.

**10.** A process according to claim 8 or claim 9, characterized in that the carboxylic acid is a saturated aliphatic acid, preferably formic acid or acetic acid, or an acid formed during the reaction, preferably pelargonic acid, azealaic acid or stearic acid.

**11.** A process according to any one of claims 8 to 10, characterized in that the quantity of carboxylic acid used is such that the molar ratio between the hydrogen peroxide and the carboxylic acid is between 10% and 500%, preferably between 10% and 40%.

**12.** A process according to any one of claims 8 to 11, characterized in that a ruthenium based catalyst is added.

**13.** A process according to any one of claims 8 to 12, characterized in that the ruthenium based catalyst is selected from: ruthenium m chloride, ruthenium IV chloride, ruthenium pentafluoride, ruthenium II oxide, ruthenium IV oxide, ammoniacal ruthenium oxychloride $Ru_2(OH)_2Cl_4$, $7NH_3$, $5H_2O$, and ruthenium acetate.

**14.** A process according to claim 12 or claim 13, characterized in that the quantity of ruthenium based catalyst used, expressed as the weight ratio between the hydrogen peroxide and the catalyst, is in the range 1% to 35%, preferably in the range 3% to 10%.

**15.** A process according to any one of claims 1 to 7, characterized in that the hydrogen peroxide is associated with a catalyst based on a metal from group Via of the periodic table, preferably a catalyst based on tungsten and/or molybdenum.

**16.** A process according to any one of claims 1 to 7 and 15, characterized in that the catalyst is preferably selected from: tungstic acid, phosphotungstic acid, molybdic acid, phosphomolybdic acid and precursors thereof.

**17.** A process according to any one of claims 1 to 7, 15 and 16, characterized in that the quantity of tungsten and/or molybdenum based catalyst used, expressed as the weight ratio between hydrogen peroxide and the catalyst. is in the range 1% to 35%, preferably in the range 3% to 10%.

**18.** a process according to claim 16 or claim 17, characterized in that a strong acid is added, preferably nitric acid, sulphuric acid or phosphoric acid.

**19.** A process according to any one of claims 1 to 18, characterized in that the temperature of the first oxidation step is in the range 30°C to 100°C, preferably in the range 60°C to 100°C.

**20.** A process according to any one of claims 1 to 19, charactenzed in that the quantity of nitric acid used in the second step represents 2 to 50 times the weight of starting substrate, preferably 4 to 10 times.

**21.** A process according to claim 20, charactenzed in that a source of $NO^-$ is added, selected from: nitrogen dioxide $NO_2$, nitrous anhydride $N_2O_3$, nitrogen peroxide $N_2O_4$, nitric oxide NO, nitrous acid, a nitrosyl or nitrose sulphate, and a nitrous salt, preferably an alkali metal salt, more preferably a sodium salt.

**22.** A process according to claim 20 or claim 21, charactenzed in that the vanadium based catalyst is selected from:

- vanadium halides such as vanadium tri-, tetra- or pentafluoride, vanadium di, tri- or tetrachloride, or vanadium tribromide;
- vanadium oxides such as vanadium oxide, vanadium dioxide, vanadium sesquioxide, or vanadium pentoxide;
- vanadium oxyhalides, in particular vanadium oxy-di- or trifluoride, vanadium oxy-mono-, di- or trichloride, or vanadium oxy-mono-, di- or tribromide;
- vanadium sulphate;
- vanadyl sulphate;
- alkali metal or ammonium vanadates in the ortho, meta or pyro forms;
- vanadyl acetylacetonate.

**23.** A process according to any one of claims 20 to 22, characterized in that the quantity of vanadium based catalyst used, expressed as the weight ratio between the catalyst expressed as $HVO_3$ and nitric acid, is preferably in the range 0.001% to 1%, more preferably in the range 0.02% to 0.5%.

**24.** A process according to any one of claims 20 to 23, charactenzed in that a co-catalyst based on a metal from groups VIIa and VIII of the periodic table is added, preferably a catalyst based on manganese, iron, nickel, ruthenium and/or cobalt.

**25.** A process according to any one of claims 20 to 24, characterized in that a nitric oxidation step is carried out in the presence of oxygen or an oxygen-containing gas.

**26.** A process according to claim 25, characterized in that the quantity of oxygen represents 1 or 2 times the stoichiometric quantity when the starting substrate is an unsaturated fatty acid and 5 to 8 times the stoichiometric quantity when the starting material is an oil or a fat.

**27.** A process according to any one of claims 20 to 26, characterized in that the reaction temperature in the second step is preferably between 40°C and 100°C, more preferably between 60°C and 90°C.

**28.** A process according to any one of claims 1 to 27, characterized in that a two-phase medium is obtained with an aqueous phase comprising the saturated dicarboxylic fatty acids, nitric acid, the catalysts and co-catalysts, and an organic phase essentially comprising the saturated monocarboxylic fatty acids formed during the reaction and optionally those present at the start, also the unsaturated fatty acids which have not reacted, and in that the saturated mono- and dicarboxylic fatty acids obtained are separated.